# EUROPÄISCHE PATENTSCHRIFT

(11) **EP 2 974 757 B1**
(45) Veröffentlichungstag und Bekanntmachung des Hinweises auf die Patenterteilung: **31.07.2019**
(21) Anmeldenummer: 15171240.3
(22) Anmeldetag: 07.12.2010
(51) Int. Cl.: A61M 1/36

(54) **SYSTEM ZUR BEFÜLLUNG UND ENTLÜFTUNG EINER VORRICHTUNG ZUR EXTRAKORPORALEN BLUTBEHANDLUNG**
SYSTEM FOR FILLING AND VENTILATING A DEVICE FOR EXTRACORPOREAL BLOOD TREATMENT
SYSTÈME DE REMPLISSAGE ET D'AÉRATION D'UN DISPOSITIF DESTINÉ AU TRAITEMENT DE SANG EXTRACORPOREL

(43) Veröffentlichungstag der Anmeldung: 20.01.2016
(62) Teilanmeldung aus: 10194070.8
(73) Patentinhaber: ZOLL LifeBridge GmbH, 84539 Ampfing (DE)
(72) Erfinder: Brieske, Gerhard, 84539 Ampfing (DE); Sagebiel, Florian, 25551 Lohbarbek (DE); Arzt, Joachim, 08468 Reichenbach (DE)
(74) Vertreter: Potter Clarkson

(56) Entgegenhaltungen:
- EP-A1- 1 457 218
- US-A1- 2002 085 952
- US-A1- 2002 176 798
- US-A1- 2008 171 960
- US-A1- 2009 076 433

## Beschreibung

### Gebiet der Erfindung

Die vorliegende Erfindung betrifft allgemein ein System zum Durchführen einer extrakorporalen Blutbehandlung. Insbesondere betriff die vorliegende Erfindung Systeme, wie Herz-Lungen-Maschinen, sowie zugehörige Vorrichtungen zur extrakorporalen Blutbehandlung, wie Patientenmodule, und damit verbundene Entlüftungsverfahren.

### Hintergrund der Erfindung

Bevor ein Einsatz einer Herz-Lungen-Maschine erfolgen kann, muss diese so vorbereitet sein, dass der Patient schnell und sicher an das System angeschlossen werden kann. Dies erfordert eine Maschine, bei der die blutführenden Komponenten durch eine Befüllflüssigkeit entlüftet werden. Lufteinschlüsse bzw. sich lösende Luftmengen können in einem späteren Einsatz am Patienten zu Luftembolien und im schlimmsten Fall zum Tod führen.

Komponenten und Schlauchsysteme herkömmlicher Herz-Lungen-Maschinen werden zur Einsatzvorbereitung zum größten Teil manuell befüllt und entlüftet. Die Vorbereitung realisiert man zum Beispiel über Teilbefüllungen von Komponenten, manuelles Klemmen von Schlauchlinien, "Ausklopfen" von Luftblasen, Kippen von Komponenten, oder zyklisches Anfahren der Blutpumpen. Komponenten wie Schläuche, Verbinder und Reservoir sind zusätzlich zu Komponenten wie Oxygenator, Filter und Blutpumpen zu entlüften.

Bei der Entlüftung durch Befüllen einer herkömmlichen Herz-Lungen-Maschine muss die Befüllung von Experten (Kardiotechniker) ausgeführt werden. Dies gilt ebenso für den laufenden Betrieb. Durch die oben beschriebenen manuellen Eingriffe wird das System für den Einsatz vorbereitet. Bei einigen Systemen läuft der Befüllvorgang halbautomatisch ab und benötigt trotzdem geschultes Personal und/oder Kardiotechniker, welche den Befüllvorgang ausführen. Bei dem halbautomatischen Verfahren muss zum Teil der Bediener Schläuche abklemmen, Pumpenaktionen starten, etc.

Generell sind diese manuellen Eingriffe aufwendig und personalkostenintensiv. Diese manuellen Eingriffe sind zudem fehleranfällig und schwierig zu protokollieren.

Die Verwendung der manuellen Eingriffe bringt insbesondere in Notfallsituationen Schwierigkeiten für den Patienten, der auf einen schnellen Einsatz der Herz-Lungen-Maschine angewiesen ist. Zudem erhöhen sich die Kosten und die Zeit, die für den Eingriff erforderlich sind.

In der Europäischen Patentschrift EP1661592B1 der Lifebridge Medizintechnik AG ist eine mobile Herz-Lungen-Maschine mit halbautomatischer Befüllung beschrieben. Diese mobile Herz-Lungen-Maschinen bestehen aus einer Basisstation und einem Steuermodul mit aufgesetztem Patientenmodul. Das Patientenmodul beinhaltet die Blut führenden Komponenten und wird nach dem Einsatz durch Trennen vom Steuermodul entsorgt. Zu den Blut führenden Komponenten zählen die Blutpumpe in Ausführung eine Zentrifugalpumpe, ein Reservoir mit Füllstandssensorik, ein arterieller Filter, ein Oxygenator, diverse Verbindungsschläuche und Bypässe sowie Sensoren zur Luft- und Gasblasenerkennung, Druck- und Flussmessung.

Bei der in EP1661592B1. beschriebenen mobilen Herz-Lungen-Maschine erfolgt das Befüllen und Entlüften der Komponenten durch ein manuelles Drehen der kompletten Einheit um 90° in eine Befüllposition. In der Befüllposition wird dann ein automatisches Verfahren gestartet, das die Befüllung ermöglicht. Nach der Befüllung wird die Einheit 90° in eine befüllte Betriebsposition zurückgedreht. Durch das Drehen in die Betriebsposition und ein Verfahren zur automatischen Luftblasenerkennung und -elimination wird das System in den Betriebszustand gebracht.

Für die Drehbewegung beim Entlüften wird jedoch eine zusätzliche mechanische Drehaufnahme benötigt, um das Steuermodul mit dem Patientenmodul von der Betriebsposition in die Befüllposition und zurück zu bewegen.

Weiterhin besteht bei den manuellen wie auch bei den halbautomatischen Systemen die Gefahr, dass trotz einer Sichtkontrolle der Komponenten Lufteinschlüsse übersehen oder nicht erkannt werden.

Ziel der Erfindung ist es somit, eine Herz-Lungen-Maschine vollautomatisch, ohne das Einwirken durch den Bediener, durch Befüllung zu Entlüften und somit für den Einsatz sicher vorzubereiten. Dabei soll zwischen dem Starten der Maschine (Initialisierung, manueller Anschluss diverser Schlauchlinien und manueller Anschluss der Befüllflüssigkeit) und dem Anschluss an den Patienten keine manuellen Eingriffe zur Entlüftung an den Komponenten im Patientenmodul stattfinden. Weiterhin soll durch einen Entfall der bisher erforderlichen Drehung des Basismoduls für den Entlüftungsvorgang die Zeit für das Entlüftungsverfahren weiter verkürzt werden. Somit steht das System für den Einsatz im Speziellen für den kurzfristigen Notfalleinsatz schnellstens zur Verfügung.

Es ist daher wünschenswert, ein Vorbereitungs-Entlüftungsverfahren einer Herz-Lungen-Maschine in Ausführung einer mobilen Herz-Lungen-Maschine bereitzustellen, welches ohne Eingriff des Bedieners stattfinden kann. Insbesondere ist es wünschenswert, dass nach dem Anschluss der Befüllflüssigkeit und dem Anschluss der Tischlinie der Befüllvorgang manuell gestartet und vollautomatisch durchlaufen werden soll.

Eine sichere Befüllmöglichkeit auch während eines Transportes erhöht die Einsatzmöglichkeiten des Systems.

Schwierig zu entlüftende Komponenten wie Blutpumpe und arterieller Filter sind vorzugsweise so bereitzustellen, dass im Entlüftungsverfahren die Luft in den Komponenten vorteilhaft entweichen kann.

Eine Dokumentation des Entlüftungsvorgangs sollte durch die Komponenten und Verfahren ermöglicht werden. Die US 2002/085952 A1 offenbart ein System zur Blutperfusion, welches einen vereinfachten Aufbau aufweist und das Verbinden/Lösen verschiedener Einwegkomponenten mit Überwachungs-/Kontrollkomponenten vereinfacht. Eine "Auto-Prime" Prozedur wird durch das Berühren einer virtuellen Taste auf einem "Auto-Prime" Bildschirm in Gang gesetzt, wodurch ein automatisches Füllen von venösen, arteriellen und kardioplegischen Flüssigkeitskreisen erfolgt. Das automatische Füllen wird auf Grundlage vorherbestimmter Protokolle gesteuert, wobei die Protokolle in einem Speicher gespeichert sind und automatische Schritte beinhalten. Das automatische Füll-Protokoll umfasst, während der Füll-Sequenz, ein mehrmaliges Umwenden des arteriellen Filters und ein mehrmaliges Zurückwenden des arteriellen Filters in die aufrechte Position, um eine Füllung und eine Entlüftung des arteriellen Filters zu ermöglichen. Die US 2002/176798 A1 offenbart ein integriertes System zur Behandlung von Blut. Die US 2009/0076433 A1 und die US 2008/0171960 A1 offenbaren jeweils ein System für einen extrakorporalen Blutkreislauf. Die EP 1 457 218 A1 offenbart eine Vorrichtung zur Blutdialyse.

### Zusammenfassung der Erfindung

Aufgabe der Erfindung ist es, ein System nach dem Oberbegriff des Anspruchs 1 mit verbesserter Entlüftung zu schaffen.

Die oben genannte Aufgabe wird durch die Merkmale des Anspruchs 1 gelöst.

Das beschriebenen Verfahren und Vorrichtungen weisen zumindest die nachstehend aufgeführten Vorteile auf.

Ein Vorbereitungs- Entlüftungsverfahren in einer Vorrichtung zur extrakorporalen Blutbehandlung, wie einer Herz-Lungen-Maschine, vorzugsweise in Ausführung einer mobilen Herz-Lungen-Maschine, kann ohne Eingriff des Bedieners statt finden. Nur Vorbereiten und Starten des Vorgangs ist notwendig. Der restliche Vorgang läuft automatisch und überwacht ab. Nach dem Anschluss der Befüllflüssigkeit und dem Anschluss der Tischlinie wird der Befüllvorgang manuell gestartet und dann vollautomatisch durchlaufen.

Das System zur extrakorporalen Blutbehandlung, wie vorzugsweise eine Herz-Lungen Maschine, kann nicht nur im stationären Bereich, sondern auch während eines Transportes befüllt werden.

Luft kann aus schwierig zu entlüftende Komponenten der Vorrichtung zur extrakorporalen Blutbehandlung, wie Blutpumpe und arterieller Filter entweichen, dank der Ausführung und Anordnung der Komponenten bzw. im Entlüftungsverfahren.

Überwachung und Aufzeichnung des Entlüftungsvorgangs ist in einem elektronischen Protokoll möglich. Die wichtigsten Zustände des Systems sind dort ablegbar. Dazu zählen z. B. der Zustand von Klemmen, Zeiten, Sensoren für Parameter wie Druck, Fluss, Luftblasendetektion.

### Kurzbeschreibung der Zeichnungen

Die Erfindung soll nachstehend anhand der in der Zeichnung dargestellten Ausführungsbeispiele näher erläutert werden.

Es zeigen:
Fig. 1 eine Querschnittszeichnung über einen arteriellen Filter in normaler Befüllrichtung;
Fig. 2 eine Querschnittszeichnung über einen arteriellen Filter in retrograder Befüllrichtung;
Fig. 3 und 4 schematische Darstellungen über eine Blutpumpe;
Fig. 5 eine schematische Übersicht über ein Kreislaufsystem zu Verfahren 1;
Fig. 6 eine schematische Übersicht über ein Kreislaufsystem für Verfahren 2 mit retrograder Befüllung;
Fig. 7 eine schematische Übersicht über ein Kreislaufsystem für Verfahren 1 mit integrierter Druckmessung;
Fig. 8 eine schematische Übersicht über ein Kreislaufsystem für Verfahren 2 mit integrierter Druckmessung;
Fig. 9a, 9b schematische Diagramme über die Schritte von diversen Verfahren; und
Fig. 10a, 10b ein schematisches Diagramm über diverse Computer Programme zur Ausführung von Verfahren.

### Ausführliche Beschreibung der Erfindung

Während die Erfindung in verschiedenen Ausführungsformen und mit der Beschreibung von verschiedenen Verfahren zum Durchführen der Erfindung beschrieben werden wird, versteht der Fachmann, dass diese Ausführungsformen nur illustrative, nicht begrenzende Beispiele von vielfältigen Formen, welche die vorliegende Erfindung einnehmen kann, darstellen. Die Vorrichtung zur extrakorporalen Blutbehandlung, ist vorzugsweise in einer Herz-Lungen Maschine angeordnet.

Genauer sind die beschriebenen Ausführungsformen der Vorrichtungen und des Systems der vorliegenden Erfindung zum Ausführen eines automatischen Entlüftungsverfahrens geeignet.

Mehrere Beispiele für solche Verfahren zum Durchführen einer Entlüftung im Zusammenhang mit der Befüllung einer Herz-Lungen-Maschine wird untenstehend im Detail beschrieben.

Wie oben erwähnt, erfolgt bei der in EP1661592B1 beschriebenen mobilen Herz-Lungen-Maschine das Befüllen und Entlüften der Komponenten durch ein manuelles Drehen der kompletten Einheit um 90° in eine Befüllposition. Nach der Befüllung wird die Einheit 90° in eine befüllte Betriebsposition zurückgedreht.

Der Befüllschritt dient der Entlüftung der im Patientenmodul befindlichen Komponenten und wird über eine 90°-Drehbewegung des Steuermoduls mit dem Patientenmodul auf der Basisstation durchgeführt. Befindet sich das Steuermodul mit dem Patientenmodul in Befüllposition, so pumpt die Blutpumpe aus einem angeschlossenen Infusionsbeutel die Befüllflüssigkeit über das Reservoir zeitgesteuert in das System. Dadurch werden die Komponenten mit der Befüllflüssigkeit gefüllt und die darin befindliche Luft ausgeleitet. Für den anschließenden Einsatz wird das Steuermodul mit dem Patientenmodul zurück in die Betriebsposition gedreht, wobei die Entlüftung der Komponenten in der Betriebsposition durch weiteres Umpumpen der Befüllflüssigkeit über eine bestimmte Zeit fortgeführt wird.

Die Drehung in die Befüllposition und somit die Entlüftung des Systems in dieser Position ist durch zwei Komponenten erforderlich, den Pumpenkopf der Blutpumpe (Zentrifugalpumpe) und den arteriellen Filter.

Der Pumpenkopf ist im System in Betriebsposition so angeordnet, dass der Auslass der Pumpe nach unten zeigt und somit möglicherweise während des Betriebes eintretende Luft im Kopf nach oben steigt, sich dort sammelt und nicht in das Kreislaufsystem gelangen kann. Würde eine Befüllung in der Betriebsposition statt finden, so könnte die Luft im oberen Bereich des Pumpenkopfes nicht entweichen. Aus diesem Grund erfolgt eine Drehung des Systems um 90° in die Befüllposition, um die Luft über den Einlass des Pumpenkopfes entweichen zu lassen und somit eine luftleere Befüllung zu gewährleisten.

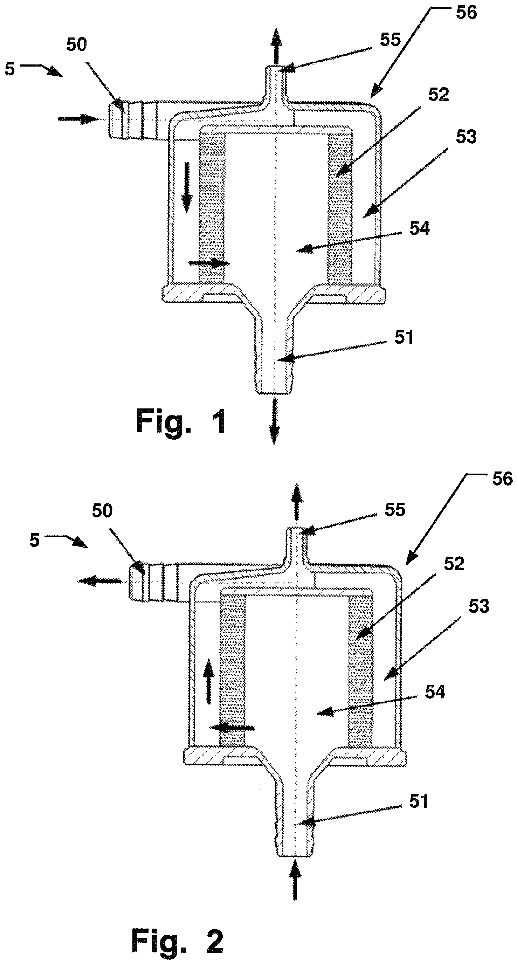

Der arterielle Filter ist im System in Betriebsposition so angeordnet, dass aufgrund seiner Bauart der Auslass nach unten zeigt und der Einlass seitlich oben am Filter angebracht ist. Würde der Filter in der Betriebsposition von der Einlassseite (ungefilterte Seite) befüllt werden, so geht die Befüllflüssigkeit von der ungefilterten Seite zur gefilterten Seite über und der Filter füllt sich. Auf der gefilterten Seite, im inneren Filterbereich, kann es zu Lufteinschlüssen im oberen, wie auch im unteren Bereich durch die Befüllung kommen. Lufteinschlüsse am unteren Auslassbereich des Filters können sich während des Betriebes lösen und somit in den Kreislauf gelangen. Wird hingegen der Filter zuerst von der oben am Filter angebrachten Entlüftungslinie (Purgeline) und dann von der Einlassseite in der Befüllposition (liegend) befüllt, so kann die Luft im Filter verdrängt werden. Die restliche im Filter verbleibende Luft wird nach der Drehung in die Betriebsposition über die Entlüftungslinie aus dem Filter entfernt, siehe Figur 1.

Ohne Drehung des Systems um 90° konnten bisher Schwierigkeiten bei der Entlüftung der Blutpumpe und des arteriellen Filters auftreten. Hier konnte es bisher vorkommen, dass sich Luftblasen in einer oder mehrerer der Komponenten ansammeln.

Beim Filter kam bisher hinzu, dass bei einer Befüllung des Filters die Filtermembranstruktur durch die Oberflächenspannung der Befüllflüssigkeit "verschlossen" wird und somit eine Entlüftung nicht möglich war. Die Luft kann nicht durch die benetzte Membran entweichen.

Auch die aufsteigende Luft aus dem am Ausgang des Filters angebrachten Schlauches sammelte sich im Auslassbereich des Filters. Der Lufteinschluss konnte durch die von der Eingangsseite nachströmenden Flüssigkeit nicht nach oben steigen und wurde immer wieder Richtung Filterauslass "mitgerissen" und zerteilt.

Prinzipiell könnte bei Drücken die höher sind als die Kapillarkräfte auch bei einer benetzten Membran Luftblasen durchgedrückt werden. Dies ist jedoch in der Praxis aus mehreren Gründen nicht durchführbar. Das Reservoir kann beispielsweise zerstört werden. Der Filter kann in Mitleidenschaft gezogen werden. Die Pumpe kann überlastet werden und ausfallen. Zudem ist ein so hoher Druck notwendig, der höher ist als der maximale Betriebsdruck des Systems. Dies führt bei zu hohen Drücken zu Leckagen. Alternativ könnte man Komponenten verwenden, die höhere Betriebsdrücke erlauben. Diese sind jedoch wesentlich teurer und somit keine Alternative im Markt.

In bestimmten Ausführungsformen der Erfindung wird der Druck am Ende der Befüllung kurzzeitig erhöht, dadurch dass die Blutpumpe mit einer erhöhten Pumpendrehzahl betrieben wird. Der Druck im System wird dabei so gewählt, dass er unterhalb, aber nahe des maximalen Betriebsdruckes liegt.

Die obengenannten Nachteile des Standes der Technik werden durch Ausführungsformen der Erfindung umgangen. Eine vollautomatische Befüllung mit Entlüftung ist somit ermöglicht ohne eine Drehung des Systems um 90°. Dies wird im Folgenden näher erläutert.

Das in Ausführungsformen beschriebene System extrakorporalen Blutbehandlung besteht aus einem Steuermodul mit aufgesetztem Patientenmodul. Im Steuermodul befinden sich die automatischen Schlauchklemmen, der Blutpumpenantrieb sowie die Auswerte- und Ansteuerelektronik. Das Patientenmodul ist eine Vorrichtung zur extrakorporalen Blutbehandlung. Im Patientenmodul befinden sich die im Blutkreislauf Blut führenden Komponenten. Das System ist somit zweiteilig aufgebaut. Die Betriebsposition (Lage) des Patientenmoduls ist auch dessen Befüllposition. Eine Drehaufnahme entfällt.

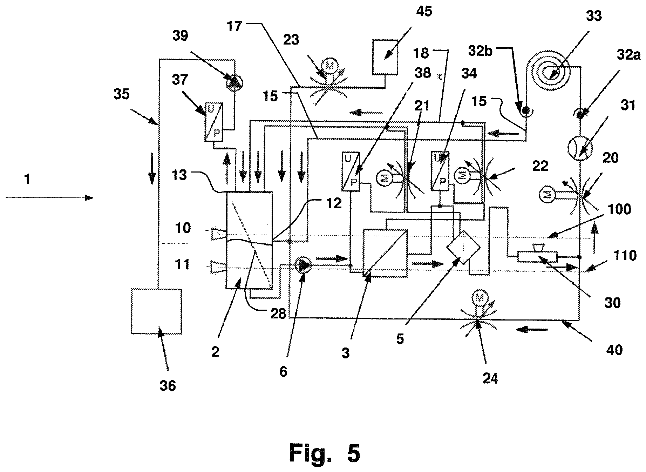

Die in Figuren 5 bis 8 dargestellten Komponenten der Patientenmodule sind, trotz der schematischen Darstellung, in Ihrer wirklichen horizontalen Lage relativ zueinander. Dies gilt insbesondere für das Reservoir 2, die Blutpumpe 6, den Oxygenator 3 und den Filter 5. Die Einzelheiten und Bedeutung der Lagen der Komponenten zueinander wird anhand von Ausführungsformen nun näher erläutert.

Flussrichtungen sind in den Diagrammen mit fett gedruckten Pfeilen an den entsprechenden Leitungen, Schläuchen oder Komponenten illustriert.

In Fig. 5 ist ein Kreislaufsystem dargestellt. Der Kreislauf ist wie folgt aufgebaut: beginnend von der venösen Seite endet die venöse Linie 15 in einem Reservoir 2 mit Screen 28. An der venösen Linie 15 befindet sich eine Volumengabelinie 17, durch die Befüllflüssigkeit aus einem Vorratsbehälter 45 und die im Einsatz möglicherweise zugeführte Flüssigkeit in den Kreislauf eingebracht werden kann. Diese Volumengabelinie 17 wird über eine dritte Schlauchklemme 23 (SKL3) hin zur venösen Linie 15 gesteuert.

Die hierin beschriebenen Schlauchklemmen sind allgemeine steuerbare Ventile für Fluidleitungen. Mit Fluid ist eine Flüssigkeit wie Blut oder Blutersatzstoff, oder ein Gas, wie Luft, gemeint.

Das Reservoir 2 besitzt einen Eingang 12 von der venösen Linie 15, eine am Reservoir-Oberteil 13 angeschlossene Rollenpumpe 39 mit Schlauchklemmfunktion zur Entlüftung des Reservoirs 2, wie im der Europäischen Patentschrift EP1705375B1 beschrieben

Das Reservoir 2 ist mit einem oberen Füllstandsensor 10 und einem unteren Füllstandsensor 11 ausgestattet. Das Reservoir weist den erwähnten Screen 28 auf, der den Einlass- und Auslassbereich über 4/5 der Fläche des Reservoirs trennt.

Das Reservoir 2 besitzt weiterhin einen Ausgang im unteren Bereich zu einer Blutpumpe 6.

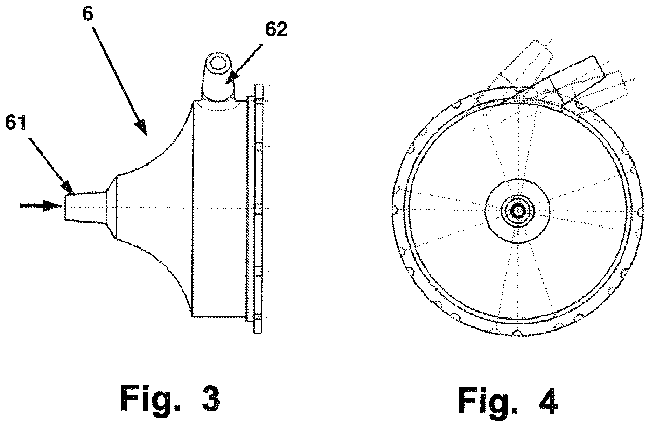

Die Blutpumpe 6 ist vorzugsweise eine Zentrifugalpumpe, wie in Fig. 3 und 4 gezeigt. Der Auslass 62 der Blutpumpe 6 ist tangential nach oben gerichtet. Der Einlass 61 der Blutpumpe 6 ist axial angeordnet. Der Pumpenkopf kann von der senkrechten Lage im Uhrzeigersinn etwa 10° und gegen den Uhrzeigersinn etwa 20° verdreht verbaut werden um den Entlüftungseffekt zu erreichen. Dies ist in Fig. 4 illustriert.

Die dem Reservoir 2 nachfolgende Blutpumpe 6 hat den axialen Einlass 61 am Reservoir 2 angeschlossen und der tangentiale Auslass 62 ist mit der venösen Seite eines Oxygenators 3 verbunden.

Von einem arteriellen Anschluss des Oxygenators 3 geht eine Flüsigkeitsleitung, wie z. B. ein Schlauch, zu einem arteriellen Filter 5. Weiterhin befinden sich am Oxygenator 3 Anschlüsse für eine Sauerstoffversorgung (nicht gezeigt), sowie Anschlüsse für ein Hyperthermiegerät zur Temperierung der Flüssigkeit im Kreislaufsystem (nicht gezeigt). Der Oxygenator 3 weist weiterhin einen Anschluss für eine Entlüftungslinie 18 auf, welche über eine zweite Schlauchklemme (SKL2) trennbar mit dem Reservoir-Oberteil verbunden ist.

Fig. 1 und 2 zeigen einen arteriellen Filter 5 in verschiedenen Zuständen. Am arteriellen Filter 5 befindet sich ebenfalls neben einem Auslass 51 zur arteriellen Seite eine am Filteroberteil angebrachte Entlüftungslinie 55 zum Reservoir-Oberteil 13, die durch eine erste Schlauchklemme 21 (SKL1) abgeklemmt werden kann. der Filter weist eine ungefilterte Seite 53 und eine gefilterte Seite 54 auf.

Nach dem arteriellen Filter ist ein Luftblasensensor 30 angeordnet, der bei Luftblasenerkennung eine nachfolgende Schnellschlussklemme 20 (SSKL) aktiviert. Eine solche SSKL 20 ist in der europäischen Patentschrift EP1698371B1 beschrieben

Die SSKL 20 schließt bei Erkennung einer Luftblase durch den Blasensensor die arterielle Linie 42 und eine vierte Schlauchklemme 24 (SKL4) für einen Bypass 40 öffnet, so dass die Flüssigkeit im Kreis umgepumpt wird, bis sich die detektierte Luftblase nicht mehr im Kreislauf des Patientenmoduls 1 befindet. Ebenso sind im Kreislauf Druck- und Flusssensoren 34, 37, 38 integriert. Vor der Entlüftung des Systems wird am venösen Anschluss 32b und am arteriellen Anschluss 32a ein Tischset 33 angeschlossen, das den Kreislauf schließt und bei der Entlüftung mit befüllt wird. Ein Patient ist während der Befüllung nicht an den Kreislauf angeschlossen.

Die Komponenten im Patientenmodul 1 sind in ihrer Lage wie folgt angeordnet.

Das Reservoir 2 ist in einer 45°-Schräglage eingebaut. Es kann aber auch senkrecht im System stehen.

Der maximale Füllstand 100 des Reservoirs 2 wird über den oberen Füllstandsensor 10 geregelt. Während des Befüllens kann somit der Flüssigkeitsstand im Patientenmodul 1 so geregelt werden, dass er sich unter der Oberseite 56 des arteriellen Filters 5 befindet. Durch diese Anordnung wird verhindert das der Filter 5 voll mit Flüssigkeit gefüllt wird und somit der obere Teil der Membran des Filterelementes 52 des Filters mit Befüllflüssigkeit benetzt wird. Lufteinschlüsse können über die noch "offenen", nicht benetzten und somit gasdurchlässigen, oberen Membranbereiche des Filters 5 passieren und entweichen.

Die Oberseite des Oxygenators 3 liegt unter oder in gleicher Höher wie die Oberseite des Filters 5. Die Blutpumpe 6 am Reservoir 2 befindet sich unterhalb des maximalen Füllstandes des Reservoirs, unter der horizontalen Lage des oberen Füllstandsensors 10. Die Blutpumpe 6 kann auch unter der unteren Kante des Reservoirs 2 angeordnet sein. Die flüssigkeitsführenden Teile der Pumpe 6 befinden sich somit immer unter dem oberen Füllstandssensor 10. Die Befüllflüssigkeit kann somit allein durch Schwerkraft vom Befüllflüssigkeitsbehältnis 45 über den tangentialen Auslass 62 der Pumpe 6 zu den nachgelagerten Komponenten gelangen. Es besteht keine Gefahr eines Lufteinschlusses in der Pumpe 6.

Das Patientenmodul 1 für eine Herz-Lungen-Maschine ist mit einer Trägerstruktur versehen, welche die räumliche Anordnung der Komponenten zueinander sicherstellt. Somit befinden sich im Patientenmodul 1 ein Blutkreislauf mit einer Blutpumpe 6, ein Oxygenator 3 mit einer Oberseite, ein Filter 5 mit einer Oberseite, und ein Reservoir 2. In Betriebsposition des Patientenmoduls sind die Komponenten in Horizontallage relativ einander in der Trägerstruktur angeordnet. Die Oberseite des Oxygenators 3 liegt unter oder in gleicher Höher wie die Oberseite des Filters 5. Die flüssigkeitsführenden Teile der Blutpumpe 6 befinden sich unter der horizontalen Lage eines oberen Füllstandsensors 10 des Reservoirs 2 und befinden sich somit unterhalb des maximalen Füllstandes des Reservoirs 2. Die Oberseite des Filters liegt höher als der obere Füllstandsensor des Reservoirs. Somit kann eine Teilbefüllung des Filters 5 gesteuert werden, welches eine vorteilhafte Entlüftung des Blutkreislaufes ermöglicht.

Der Filter 5 des Patientenmoduls hat in einer Ausführungsform eine an der Oberseite angeschlossene Entlüftungslinie, wobei ein Anschlusspunkt für die Entlüftungslinie höher liegt als der obere Füllstandsensor 10.

Das erste steuerbare Ventil 21 (SKL1) ist in der Entlüftungslinie des Filters angeordnet.

Eine Steuereinheit 302 der Herz-Lungen-Maschine ist so konfiguriert, dass nach dem Ansprechen des oberen Füllstandssensors 10 das erste steuerbare Ventil 21 (SKL1) zumindest teilweise geschlossen wird.

Die untere Kante des Reservoirs 2 weist eine untere Kante auf, und die Blutpumpe 6 liegt unter der unteren Kante oder unter einem Horizontalniveau der unteren Kante des Reservoirs (nicht gezeigt). Alternativ, liegt die Blutpumpe 6 in der Höhe des unteren Drittels des Reservoirs.

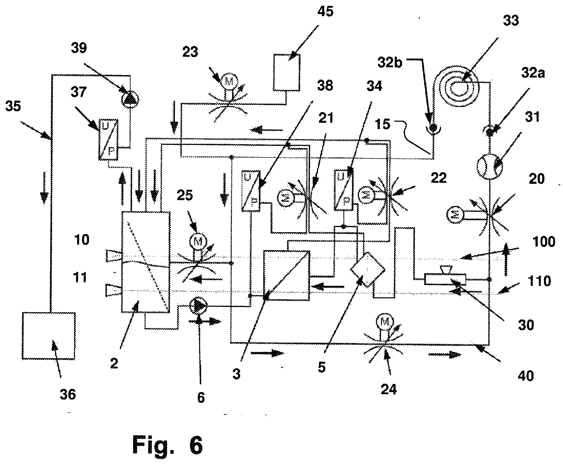

Das Patientenmodul kann eine zusätzliche fünfte Schlauchklemme 25 (SKL5) aufweisen. Die SKL5 25 ist in der venösen Linie 15 zwischen Volumengabeeinlass und Reservoir 2 und einer Einleitungsstelle der Befüllflüssigkeit, die höher als das Reservoir, der Filter und der Oxygenator liegt, angeordnet, siehe die Ausführungsform des Patientenmoduls la in Fig. 6 dargestellt.

Die Herz-Lungen-Maschine weist eine Steuereinheit zur vollautomatischen Befüllung und Entlüftung eines erfindungsgemäßen Patientenmoduls auf. Die Steuereinheit ist, zur Befüllung und Entlüftung des Patientenmoduls, konfiguriert das erste steuerbare Ventil 21 (SKL1), welches nach Start des Befüllvorgangs für die Entlüftungslinie des Filters 5 geöffnet ist, und nach Ansprechen des oberen Füllstandssensors 10 im Reservoir 2 zu schließen um die aktive Befüllung des Filters 5 zu steuern. Aus dem höher als das Patientenmodul angeordnetem und mit diesem verbundenen Befüllflüssigkeitsbehältnis 45 strömt somit die Befüllflüssigkeit durch Schwerkraft über die venöse Seite des Systems in das Reservoir 2 und weiter in die am unteren Ende des Reservoirs 2 befindliche Blutpumpe 6 und dann weiter in den Filter 5.

Somit wird durch das geschlossene erste steuerbare Ventil 21 (SKL1) und die somit geschlossene Entlüftungslinie und ein Luftpolster im Filter geschaffen. Das Luftpolster dämpft die einströmende Befüllflüssigkeit in ihrem Strömungsverhalten. Eine erste Drehzahl der Blutpumpe wird von der Steuereinheit 302 so gewählt, dass eine langsame Befüllung des Filters 5 von der Einlassseite erfolgt und dieser nur zum Teil befüllt wird und ein oberer Bereich der Filtermembran 52 von der Befüllflüssigkeit unbenetzt bleibt und somit für den weiteren Befüll- und Entlüftungsvorgang luftdurchlässig bleibt.

Weiterhin ist die Steuereinheit in Ausführungsformen, wie in Fig. 6, konfiguriert, bei Erkennung der Befüllflüssigkeit im Reservoir durch den unteren Füllstandssensor 11 das fünfte steuerbare Ventil 25 (SKL5) zu schließen. Dadurch wird mittels des fünften steuerbaren Ventils 25 (SKL5) eine venöse Verbindung zwischen Volumengabeeinlass und dem Reservoir 1 getrennt. Die Befüllflüssigkeit wird nun nur noch durch die venöse Linie über den Bypass 40 zum arteriellen Filter 5 hin abfließen. Damit wird durch den anliegenden Druck über die Schwerkraft der Filter 5 von der Auslassseite retrograd befüllt.

Das Patientenmodul kann einen Luftblasensensor 30 umfassen. Der Luftblasensensor ist angeordnet, um Luft im System zu erkennen. Die Steuereinheit ist konfiguriert bei einer Luftblasenerkennung durch den Sensor mittels der Schnellschlussklemme SSKL 20 das Tischset wegzuschalten. Das vierte steuerbare Ventil 24 (SKL4) wird wieder geöffnet und die Luft im System wird über das Reservoir abgeführt.

Der Befüll- und Entlüftungsvorgang ist mittels des Patientenmoduls in der Herz-Lungen-Maschine während eines Transportes durchführbar.

Die Steuereinheit 302 ist angepasst, die Entlüftung vollautomatisch durchzuführen und nach dem Start des Entlüftungsvorgangs alle Abläufe automatisch auszuführen bis der Entlüftungsvorgang abgeschlossen ist.

Nach dem Start wird die Volumengabelinie durch das dritte steuerbares Ventil 23 (SKL3) geöffnet und dabei das zweite steuerbare Ventil SKL2 22, das vierte steuerbare Ventil 24 SKL4 und die Schnellschlussklemme SSKL 20, sowie die Rollenpumpe 39 mit Schlauchklemmfunktion geöffnet sind.

Die Herz-Lungen-Maschine ist in Ausführungsformen dazu angepasst, den Vorgang der Befüllung und Entlüftung aufzuzeichnen und somit auslesbar zu protokollieren. Beispielsweise könne Daten, wie die Schaltstellungen der steuerbaren Ventile, Füllstände des Reservoirs, Aus- und Einschaltzeiten, das Ansprechen des Luftblasensensors und weitere Parameter wie Druck und Fluss in einer Speichereinheit abgelegt werden. Die Steuereinheit 302 kann diese Daten von und zur Speichereinheit administrieren.

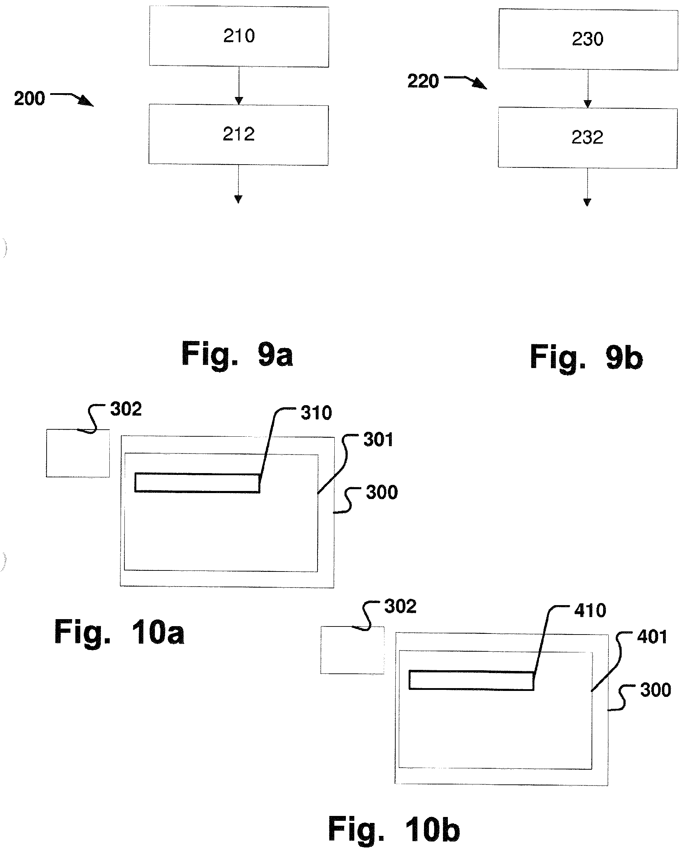

Ein Computerprogramm 301 zur Steuerung der Befüllung und Entlüftung eines Patientenmoduls ist in Fig. 10a dargestellt. In einer Herz-Lungen-Maschine, ohne daran angeschlossenen Patienten, wird das Computer Programm 301 durch eine Recheneinheit, wie die Steuereinheit 302 ausgeführt. Das Computer Programm ist auf einem Computer lesbaren Speichereinheit implementiert. Während der genannten Befüllung und Entlüftung wird aus einem höher als das Patientenmodul befindlichen Befüllflüssigkeitsbehältnis eine Befüllflüssigkeit durch Schwerkraft über eine venöse Seite des Systems in ein Reservoir geströmt und weiter in eine am unteren Ende des Reservoirs befindliche Blutpumpe geströmt. Das Computerprogramm umfasst Kodsegmente, wobei es ein Kodsegment 310 umfasst, dass ein erstes steuerbares Ventil (SKL1) für eine Entlüftungslinie eines Filters geöffnet wird und nach dem Ansprechen eines oberen Füllstandssensors im Reservoir geschlossen wird. Das Computerprogramm 301 ist vorzugsweise zur Ausführung der untenstehenden Ausführungsform eines "ersten Verfahrens" geeignet.

Ein Kit für eine Herz-Lungen-Maschine umfasst ein erfindungsgemäßes Patientenmodul, ein Tischset 33, und ein Befüllflüssigkeitsbehältnis 45 für eine Befüllflüssigkeit.

Ein weiteres Computerprogramm 401 zur Steuerung der Befüllung und Entlüftung eines Patientenmoduls ist in Fig. 10b dargestellt. In einer Herz-Lungen-Maschine, ohne daran angeschlossenen Patienten, wird das Computer Programm durch eine Recheneinheit, wie die Steuereinheit 302 ausgeführt. Das Computer Programm 401 ist auf einem Computer lesbaren Speichereinheit implementiert. Während der genannten Befüllung und Entlüftung wird aus einem höher als das Patientenmodul befindlichen Befüllflüssigkeitsbehältnis eine Befüllflüssigkeit durch Schwerkraft über eine venöse Seite des Systems in ein Reservoir geströmt und weiter in eine am unteren Ende des Reservoirs befindliche Blutpumpe geströmt. Die Blutpumpe befindet sich unterhalb eines unteren Füllstandsensors des Reservoirs. Das Computerprogramm umfasst Kodsegmente, wobei es ein Kodsegment 410 umfasst, welches bei Erkennung der Befüllflüssigkeit im Reservoir durch den unteren Füllstandssensor das fünfte steuerbare Ventil (SKL5) schließt und wodurch mittels des fünften steuerbaren Ventils (SKL5) eine venöse Verbindung zwischen Volumengabeeinlass und dem Reservoir getrennt wird und die Befüllflüssigkeit nur noch durch die venöse Linie über einen Bypass zu einem arteriellen Filter hin abfließt, und womit durch den anliegenden Druck über die Schwerkraft der Filter von der Auslassseite retrograd befüllt wird. Das Computerprogramm 401 ist vorzugsweise zur Ausführung der untenstehenden Ausführungsform eines "zweiten Verfahrens" geeignet.

Fig. 9a zeigt ein schematisches Diagramm über die Schritte eines ersten Verfahrens und Fig. 10a ein schematisches Diagramm über ein Computer Programm zur Ausführung des Verfahrens.

In Fig. 5 ist das oben erläuterte Kreislaufsystem zu dem ersten Verfahren 200, wie in Fig. 9a gezeigt, dargestellt. Diese Verfahren 200 ist ein Verfahren zur Befüllung und Entlüftung eines Patientenmoduls in einer Herz-Lungen-Maschine. Ein Patient ist während des Befüllens nicht daran angeschlossen. Das Verfahren umfasst den Schritt 210, wobei aus einem höher als das Patientenmodul befindlichen Befüllflüssigkeitsbehältnis eine Befüllflüssigkeit durch Schwerkraft über eine venöse Seite des Systems in ein Reservoir strömt und weiter in eine am unteren Ende des Reservoirs befindliche Blutpumpe strömt. Das erste steuerbare Ventil 21 SKL1 ist für die Entlüftungslinie des Filters 5 geöffnet. In einem weiteren Schritt 212 wird nach dem Ansprechen eines oberen Füllstandssensors im Reservoir das erste steuerbare Ventil geschlossen.

Nach dem Start des Entlüftungsvorgangs laufen alle Prozesschritte automatisch ab bis der Entlüftungsvorgang abgeschlossen ist. Nach dem Start öffnet die SKL3 die Volumengabelinie. Aus dem höher als das Patientenmodul befindlichen Befüllflüssigkeitsbehältnis 45 strömt die Befüllflüssigkeit durch Schwerkraft über die venöse Seite des Systems in das Reservoir 2 und weiter in die am unteren Ende des Reservoirs 2 befindliche Blutpumpe 6 in Ausführung einer Zentrifugalpumpe. Dabei sind die SKL2, SKL4 und die SSKL sowie die Rollenpumpe 39 mit Schlauchklemmfunktion geöffnet. Die SKL1 für die Entlüftungslinie des Filters 5 ist ebenfalls geöffnet und wird erst nach dem Ansprechen des oberen Füllstandssensors 10 im Reservoir geschlossen. Ebenfalls wird nach dem Ansprechen des oberen Füllstandssensors 10 im Reservoir die Rollenpumpe 39 geschlossen somit kann über den Entlüftungsweg 35 zum Auffangbeutel 36 keine Luft mehr entweichen und die Befüllung des Reservoirs 2 über das Befüllflüssigkeitsbehältnis 45 wird gestoppt. Je nach Befüllzustand des Reservoirs 2 wird die Rollenpumpe 39 geöffnet oder geschlossen. Erreicht die Befüllflüssigkeit den oberen Füllstandssensors 10 im Reservoir 2 so geht man davon aus, dass die nachgelagerten Komponenten mit Befüllflüssigkeit durch die Schwerkraft gefüllt sind. Die SKL1 wird nach Erreichen des oberen Füllstandsensors 10 geschlossen, um die weitere aktive Befüllung des Filters 5 zu gewährleisten Ein vorteilhaftes Luftpolster entsteht.

Durch die Bauweise von Zentrifugalpumpen muss in diesen zur aktiven Förderung von Flüssigkeiten eine Flüssigkeit in der Pumpe vorhanden sein. Ein Ansaugverhalten bei Luft im Pumpenkopf, wie einer Membranpumpe, besteht nicht.

Die im Patientenmodul befindliche Blutpumpe 6 ist in ihrer Einbaulage so ausgerichtet ist, dass sie durch den nach oben gerichteten tangentialen Auslass 62 durch den axialen Einlass 61 über Schwerkraft mit Befüllflüssigkeit befüllt werden kann, siehe Figur 3. Die Luft steigt in der Blutpumpe 6 nach oben und wird über den Auslass 62 abgeführt.

Nach erfolgter Schwerkraftbefüllung wird durch eine Start-Stopp-Bewegung der Pumpe 6, die in der Blutpumpe befindliche Luft zum Auslass 62 transportiert und abgeführt. Während eines Stopps der Pumpe kann die Luft Richtung Pumpenauslass 62 steigen und durch das Starten der Blutpumpe 6 abtransportiert werden.

Die Anordnung von Reservoir 2 und Blutpumpe 6, nämlich unter dem maximalen Füllstand des Reservoirs 2, gewährleistet, dass die Blutpumpe 6 über Schwerkraft befüllt wird und somit immer Flüssigkeit während des Entlüftungsvorgangs in der Blutpumpe 6 steht.

Die mit Befüllflüssigkeit gefüllte Blutpumpe 6 wird in periodischen Abständen mit einer geringen Drehzahl, je nach Ausführungsform und Fördermenge, betrieben. Die Befüllflüssigkeit wird aus dem Reservoir 45 in den Oxygenator 3 und weiter in den arteriellen Filter 5 gefördert. Durch die langsame Befüllung des Filters 5 von der Einlassseite 50 wird dieser nur zum Teil befüllt. Der obere Bereich der Filtermembran 52 bleibt von der Befüllflüssigkeit unbenetzt und ist somit weiter luftdurchlässig. Eventuelle Lufteinschlüsse können von der gefilterten Seite 54 zur ungefilterten Seite 53 übertreten und abgeführt werden. Die eingebrachte Befüllflüssigkeit geht durch den unteren Teil der Filtermembran zur gefilterten Ausgangseite 54 und füllt das nachgelagerte Kreislaufsystem. Die Luft aus dem an der Filterausgangsseite 54 angeschlossenen Schlauches kann in den Filter 5 und auf die ungefilterte Seite 53 durch den unbenetzten Teil der Filtermembran 52 entweichen.

Weiterhin entsteht durch die geschlossene Entlüftungslinie, da die SKL1 geschlossen ist, und die am Filterausgang befindliche Befüllflüssigkeit ein Luftpolster im Filter 5. Das Luftpolster dämpft die einströmende Befüllflüssigkeit in ihrem Strömungsverhalten auf vorteilhafte Weise.

Nach einer festgelegten Befüllzeit mit geringer Drehzahl und somit Fördermenge wird die Drehzahl der Blutpumpe 6 schrittweise bis auf einen konstanten Wert erhöht und der Bypass 40 durch Schließen der Schlauchklemme SKL4 geklemmt und somit das angeschlossene Tischset 33 durchströmt und entlüftet.

Im folgenden Schritt wird die SKL4 wieder geöffnet und die Drehzahl der Blutpumpe 6 weiter erhöht. Durch das nochmalige Schließen der SKL4 und eine weitere Erhöhung der Drehzahl und somit der Erhöhung der Förderleistung der Blutpumpe 6 wird das Tischset 33 stärker durchströmt und weiter entlüftet. Während dessen erfolgt ein Öffnen der SKL1, d.h. der Entlüftungsklemme des arteriellen Filters 5. Die im oberen Teil des Filters verbliebene Luft kann somit entweichen.

Um den Bypass nochmals zu entlüften, wird die SKL4 wieder geöffnet. Sollte bei den letzten Schritten durch den Luftblasensensor 30 Luft im System erkannt werden, so klemmt die SSKL das Tischset 33 ab, die SKL4 öffnet sich und die Luft im System kann über das Reservoir 2 abgeführt werden. Durch die Füllstandssensorik im Reservoir 2 wird der Flüssigkeitspegel permanent überwacht, um dem System genügend Befüllflüssigkeit zur Verfügung zu stellen.

Der Vorgang der Entlüftung wird aufgezeichnet und somit auslesbar protokolliert. Es können die Schaltstellungen der Schlauchklemmen, Füllstände des Reservoirs, Aus- und Einschaltzeiten, das Ansprechen des Luftblasensensors und weitere Parameter wie Druck und Fluss abgelegt werden.

Der Entlüftungsvorgang kann auch während eines Transportes oder mobilen Einsatzes der Herz-Lungen-Maschine durchgeführt werden.

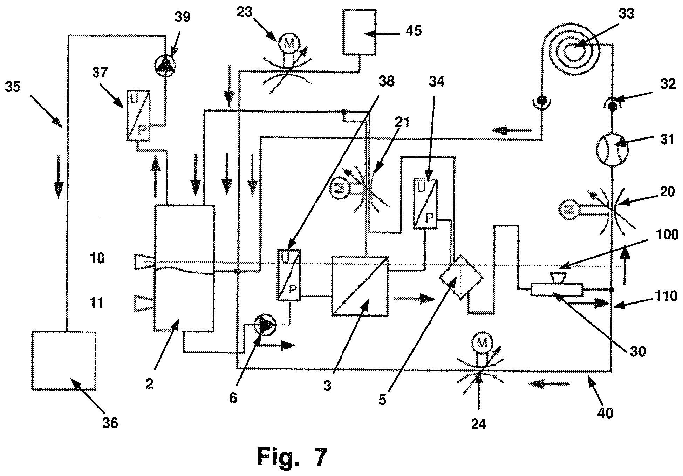

Fig. 7 zeigt eine schematische Übersicht über ein Kreislaufsystem für das erste Verfahren 200 mit integrierter Druckmessung. Das Kreislaufsystem ist wie das für das bisher erläuterte erste Verfahren, jedoch mit vier steuerbaren Ventilen, hier Schlauchklemmen. Die SKL1 und SKL2 sind zu einer Schlauchklemme durch Integration der Druckmessung in das Schlauchsystem zwischen Blutpumpe 6 und Oxygenator 3 sowie Oxygenator 3 und Filter 5 zusammengefasst.

Fig. 6 ist eine schematische Übersicht über ein Kreislaufsystem für ein zweites Verfahren 220 mit retrograder Befüllung. Fig. 9b ist ein schematisches Diagramm über die Schritte des zweiten Verfahrens 220. Fig. 10b ein schematisches Diagramm über ein Computer Programm 401 zur Ausführung eines zweiten Verfahrens.

Die Voraussetzungen sind wie im ersten Verfahren, nur mit der Blutpumpe 6 am Reservoir 2, die sich unterhalb des unteren Füllstandsensors 11 des Reservoirs 2 befindet. Die Blutpumpe 6 kann auch unter der unteren Kante des Reservoirs 2 angeordnet sein. Eine zusätzliches steuerbares Ventil, hier die Schlauchklemme SKL5 ist in der venösen Linie zwischen Volumengabeeinlass und Reservoir 2 und einer Einleitungsstelle der Befüllflüssigkeit, die höher als das Reservoir, der Filter und der Oxygenator liegt, angeordnet.

Das zweite Verfahren 220 ist ein Verfahren zur Befüllung und Entlüftung eines Patientenmoduls in einer Herz-Lungen-Maschine, ohne daran angeschlossenen Patienten. Aus einem höher als das Patientenmodul befindlichen Befüllflüssigkeitsbehältnis strömt eine Befüllflüssigkeit durch Schwerkraft über eine venöse Seite des Patientenmoduls in das Reservoir 2 und weiter in die am unteren Ende des Reservoirs 2 befindliche Blutpumpe 6. Die Blutpumpe befindet sich unterhalb des unteren Füllstandsensors 11 des Reservoirs 2.

Bei Erkennung der Befüllflüssigkeit im Reservoir 2 wird durch den unteren Füllstandssensor 11 das fünfte steuerbare Ventil 25 (SKL5) geschlossen. Mittels des fünften steuerbaren Ventils 25 wird somit eine venöse Verbindung zwischen Volumengabeeinlass und dem Reservoir 2 getrennt. Die Befüllflüssigkeit fließt nur noch durch die venöse Linie über den Bypass 40 zum arteriellen Filter 5 hin ab. Durch den anliegenden Druck über die Schwerkraft wird der Filter von der Auslassseite 51 retrograd befüllt, siehe Fig. 2.

Nach dem Start des Entlüftungsvorgangs werden alle Abläufe automatisch ausgeführt bis der Entlüftungsvorgang abgeschlossen ist. Nach dem Start öffnet die SKL3 die Volumengabelinie. Aus dem höher als das Patientenmodul befindlichen Befüllflüssigkeitsbehältnis 45 strömt die Befüllflüssigkeit durch Schwerkraft über die venöse Seite des Systems in das Reservoir 2 und weiter in die am unteren Ende des Reservoirs befindliche Blutpumpe 6 in Ausführung eine Zentrifugalpumpe. Dabei sind die SKL1, SKL2, SKL4, SKL5 und die SSKL sowie die Rollenpumpe mit Schlauchklemmfunktion geöffnet.

Die Befüllflüssigkeit strömt über die venöse Linie durch Schwerkraft gleichzeitig in das Reservoir 2 und über den Bypass 40 in Richtung Ausgang des arteriellen Filters 5. Der Filter 5 kann aufgrund seiner erhöhten Einbaulage nicht gefüllt werden, da die Befüllflüssigkeit in das Reservoir 2 abfließt.

Die im Patientenmodul befindliche Blutpumpe 6 ist in ihrer Einbaulage so ausgerichtet, dass sie durch den nach oben gerichteten tangentialen Auslass 62 durch den axialen Einlass 61 über Schwerkraft mit Befüllflüssigkeit befüllt werden kann, siehe Figur 3. Die Luft steigt in der Blutpumpe 6 nach oben und wird über den Auslass 62 abgeführt.

Sobald die Befüllflüssigkeit im Reservoir 2 den unteren Füllstandssensor 11 im Reservoir erreicht hat, ist die nachgeschaltete Blutpumpe vollständig mit Befüllflüssigkeit gefüllt.

Der untere Füllstandssensor 11 erkennt die Befüllflüssigkeit im Reservoir und die SKL5 wird geschlossen. Somit ist die venöse Verbindung zwischen Volumengabeeinlass und dem Reservoir 2 getrennt. Die Befüllflüssigkeit kann nur noch durch die venöse Linie über den Bypass 40 zum Filter 5 hin abfließen. Durch den anliegenden Druck über die Schwerkraft, wird der Filter 5 von der Auslassseite retrograd befüllt. Dadurch kann im Filter die Luft von der gefilterten Seite zur ungefilterten Einlassseite entweichen, siehe Figur 2. Aufgrund der Befüllung durch die Schwerkraft erfolgt eine allmähliche Entlüftung und Befüllung des Filters 5. Die Luft kann über die Entlüftungslinie am Filter-Oberteil entweichen und die Befüllflüssigkeit weiter zum Oxygenator 3 strömen. Im Oxygenator 3 erfolgt ebenfalls eine retrograde Befüllung. Die Luft wird durch die Entlüftungslinie des Oxygenators abgeleitet.

Im nächsten Schritt erfolgt das Öffnen der SKL5 und die Befüllflüssigkeit strömt wieder in das Reservoir 2 bis der obere Füllstandsensor 10 Flüssigkeit erkennt. Dadurch wird die Blutpumpe 6 aktiviert und deren Drehzahl schrittweise bis auf einen konstanten Wert erhöht. Weiterhin erfolgt ein Abklemmen des Bypasses 40 durch Schließen der Schlauchklemme SKL4 und ein Durchströmen des angeschlossenen Tischsets 33.

Im folgenden Schritt wird die SKL4 wieder geöffnet und die Drehzahl der Blutpumpe 6 weiter erhöht. Durch das nochmalige Schließen der SKL4 und eine weitere Erhöhung der Drehzahl und somit der Erhöhung der Förderleistung der Blutpumpe 6 wird das Tischset 33 stärker durchströmt und entlüftet. Die geöffneten Entlüftungslinien des Filters 5 und des Oxygenators, durch die SKL1 und SKL2 lassen vorhandene Luft in den Komponenten hin zum Reservoir 2 entweichen.

Durch das Schließen der SKL4 und SSKL werden im letzten Schritt der Entlüftung, die Entlüftungslinien des Oxygenators 3 und des arteriellen Filters 5 nochmals gespült.

Sollte bei den letzten Schritten durch den Luftblasensensor 30 Luft im System erkannt werden, so klemmt die SSKL das Tischset ab, die SKL4 öffnet sich und die Luft im System kann über das Reservoir 2 abgeführt werden.

Durch die Füllstandssensorik im Reservoir 2 wird der Flüssigkeitspegel permanent überwacht, um dem System genügend Befüllflüssigkeit zur Verfügung zu stellen.

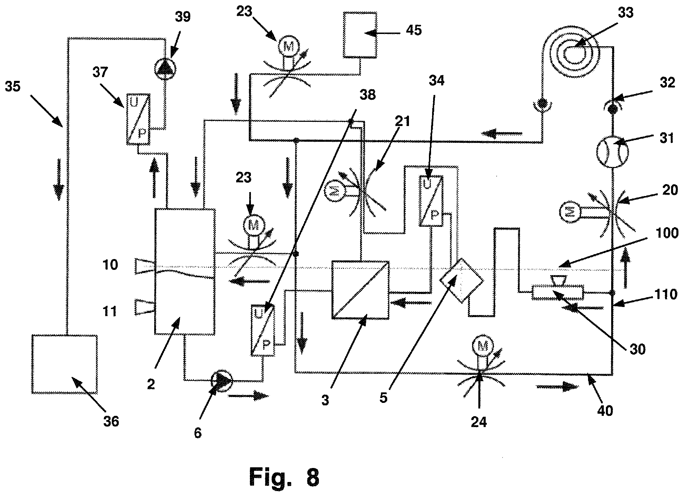

Fig. 8 ist eine schematische Übersicht über ein Kreislaufsystem für das zweite Verfahren mit integrierter Druckmessung.

Das Kreislaufsystem ist wie in Fig. 6 für das zweite Verfahren 220 beschrieben aufgebaut, jedoch mit nur fünf Schlauchklemmen. Die SKL1 und SKL2 sind zu einer Schlauchklemme durch Integration der Druckmessung in das Schlauchsystem zwischen Blutpumpe 6 und Oxygenator 3, sowie Oxygenator 3 und Filter 5, zusammengefasst. Die Blutpumpe 6 ist unterhalb der Unterkante des Reservoirs angeordnet.

Der Vorgang der Entlüftung wird aufgezeichnet und somit auslesbar protokolliert. Es können die Schaltstellungen der Schlauchklemmen, Füllstände des Reservoirs, Aus- und Einschaltzeiten, das Ansprechen des Luftblasensensors und weitere Parameter wie Druck und Fluss abgelegt werden.

Der Entlüftungsvorgang kann auch während eines Transportes oder mobilen Einsatzes der Herz-Lungen-Maschine durchgeführt werden.

Der Patienten wird nach der Befüllung und Entlüftung wie folgt angeschlossen. Während der Befüllung wird der Patient für den Einsatz vorbereitet, hierbei wird eine arterielle Kanüle in die Fermoral-Arterie und eine venöse Kanüle in die Fermoral-Vene des Patienten eingeführt. Nach der Befüllung des Systems wird das mit dem System befüllte Tischset in der Mitte getrennt (durchgeschnitten) und ohne Lufteinschlüsse an die vorbereiteten Kanülen gesteckt und mit der Perfusion begonnen.

Eine Reihe medizinischer Verfahren ist dann am Patienten durchführbar. Beispiele für medizinische Anwendungsfälle sind u.a. ein Notfalleinsatz bei akutem Herzkreislaufversagen (kardiogener Schock); Herzunterstützung zur Vermeidung von Organschäden; Lungenversagen (ARDS); Hochrisikoeingriffe am Herzen (PCI [Stent], DAVI [Herzklappen]); Unterstützung bei Beating Heart Chirurgie (CABG [Bypass OP am schlagenden Herzen]); Perfusion von Spenderkanditaten für die Organtransplantation; Temperaturstabilsierung von unterkühlten Patienten (Erwärmung des Patienten auf Körpertemperatur); Temperierung des Patienten => Hypothermie, gewollte Absenkung der Körpertemperatur z. B. Apoplex.

Der Fachmann wird nachvollziehen können, dass Änderungen und Modifikationen der beschriebenen Beispiele möglich sind, ohne vom Umfang der beigefügten Ansprüche abzuweichen.

**Bezugszeichenliste**

| | |
|---|---|
| 5 Filter | 23 Schlauchklemme 3 SKL 3 |
| 51 Auslass | 24 Schlauchklemme 4 SKL 4 |
| 52 Filterelement | 25 Schlauchklemme 5 SKL 5 |
| 53 ungefilterte Seite | |
| 54 gefilterte Seite | 28 Screen in Reservoir 2 |
| 55 Entlüftungsanschluss | |
| 56 Oberseite Filter | 30 Blasensensor |
| | 31 Flussmesser |
| 6 Blutpumpe | 32a,b Verbinder |
| 61 axialer Einlass | 33 Tischset |
| 62 tangentialer Auslass | 34 Druckmesser |
| | 35 Entlüftungslinie |
| 1, 1a Patientenmodul | 36 Auffangbeutel |
| 10 oberer Füllstandssensor | 37 Druckmesser |
| 100 Lage oberer Füllstandssensor | 38 Druckmesser |
| | 39 Rollenpumpe |
| 11 unterer Füllstandssensor | |
| 110 Lage unterer | 40 Bypass |
| Füllstandssensor | 42 arterielle Linie |
| 12 Eingang zum von venöser | |
| Linie zu Reservoir 2 | 45 Vorratsbehälter |
| 13 Reservoir-Oberteil | |
| | 200 Verfahren 1 |
| 15 venöse Linie | 210, 212 Verfahrensschritte |
| 17 Volumengabelinie | |
| 18 Entlüftungslinie Oxygenator | 220 Verfahren 1 |
| | 230, 232 Verfahrensschritte |
| | |
| 2 Reservoir | 300 Speichermedium |
| 3 Oxygenator | 301, 401 Computerprogramm |
| | 302 Prozessor, Steuereinheit |
| 20 Schnellschlauchklemme SSKL | 310, 410 Programmsegmente |
| 21 Schlauchklemme 1 SKL 1 | |
| 22 Schlauchklemme 2 SKL 2 | |

## Patentansprüche

1. System zur extrakorporalen Blutbehandlung, das zweiteilig aufgebaut ist und aus einem Steuermodul mit aufgesetztem Patientenmodul besteht, wobei:
das Steuermodul automatische Schlauchklemmen oder steuerbare Ventile für Fluidleitungen, einen Blutpumpenantrieb sowie eine Auswerte- und Ansteuerelektronik (302) aufweist;
das Patientenmodul (1, 1a), das eine Vorrichtung zur extrakorporalen Blutbehandlung ist, einen Blutkreislauf aufweist, der eine venöse Linie (15), ein Reservoir (2), einen Oxygenator (3), einen Luftblasendetektor (30), einen Filter (5), eine arterielle Linie (42) und eine Blutpumpe (6) umfasst;
das Reservoir (2) mit einem oberen Füllstandsensor (10) ausgestattet ist;
der Filter (5) ein Filterelement umfasst und der Filter eine an einem Filteroberteil angebrachte Entlüftungslinie aufweist, wobei die Entlüftungslinie durch ein erstes steuerbares Ventil (21), welches in der Entlüftungslinie angeordnet ist, geschlossen werden kann;
das System eine Steuereinheit (302) zur vollautomatischen Befüllung und Entlüftung eines erfindungsgemäßen Patientenmoduls aufweist;
eine Oberseite des Filters (5) höher liegt als der obere Füllstandsensor (10) des Reservoirs und somit eine Teilbefüllung des Filters (5) gesteuert werden kann;
die Steuereinheit, zur Befüllung und Entlüftung des Patientenmoduls, konfiguriert ist das erste steuerbare Ventil (21), welches nach Start des Befüllvorgangs für die Entlüftungslinie des Filters (5) geöffnet ist, nach Ansprechen des oberen Füllstandssensors (10) im Reservoir (2) zu schließen um eine aktive Befüllung des Filters (5) zu steuern; und
durch das geschlossene erste steuerbare Ventil (21) und die somit geschlossene Entlüftungslinie ein Luftpolster im Filter geschaffen wird, welches einströmende Befüllflüssigkeit in ihrem Strömungsverhalten derart dämpft, dass der Filter nur zum Teil befüllt wird und ein oberer Bereich des Filterelements von der Befüllflüssigkeit unbenetzt bleibt.

2. System nach Anspruch 1, wobei das System dazu angepasst, den Vorgang der Befüllung und Entlüftung aufzuzeichnen und somit auslesbar zu protokollieren.

3. System nach Anspruch 2, wobei das System eine Speichereinheit aufweist in der Daten wie die Schaltstellungen der steuerbaren Ventile, Füllstände des Reservoirs, Aus- und Einschaltzeiten, das Ansprechen des Luftblasensensors und weitere Parameter wie Druck und Fluss abgelegt werden können.

4. System nach Anspruch 2 oder 3, wobei das System ferner Druck- und Flusssensoren umfasst, welche in den Blutkreislauf integriert sind.

5. System nach Anspruch 1,wobei die Blutpumpe unter dem maximalen Füllstand des Reservoirs 2 angeordnet ist, so dass die Blutpumpe 6 über Schwerkraft befüllt werden kann und somit immer Flüssigkeit während des Entlüftungsvorgangs in der Blutpumpe 6 steht.

6. System nach Anspruch 1, wobei die Blutpumpe (6) eine Zentrifugalpumpe ist.

7. System nach Anspruch 1, wobei: der Luftblasensensor (30) nach dem Filter (5) angeordnet ist und bei Luftblasenerkennung eine nachfolgende Schnellschlussklemme (20) aktiviert, wobei die Schnellschlussklemme (20) bei Erkennung einer Luftblase durch den Luftblasensensor eine arterielle Linie (42) schließt und eine Schlauchklemme (24) für einen Bypass (40) öffnet, so dass die Flüssigkeit im Kreis umgepumpt wird, bis sich die detektierte Luftblase nicht mehr im Blutkreislauf des Patientenmoduls (1) befindet.

## Claims

1. System for extracorporeal blood treatment, which is constructed in two parts and consists of a control module comprising an attached patient module, wherein:
the control module has automatic hose clips or controllable valves for fluid lines, a blood pump drive and an evaluation and control electronic system (302);
the patient module (1, 1a) which is a device for extracorporeal blood treatment has a blood circulation system which comprises a venous line (15), a reservoir (2), an oxygenator (3), an air bubble detector (30), a filter (5), an arterial line (42) and a blood pump (6);
the reservoir (2) is equipped with an upper filling level sensor (10);
the filter (5) comprises a filter element and the filter has a venting line attached to an upper filter part, the venting line being closable by means of a first controllable valve (21) which is arranged in the venting line;
the system has a control unit (302) for the fully automatic filling and venting of a patient module according to the invention;
an upper face of the filter (5) is higher than the upper filling level sensor (10) of the reservoir and thus partial filling of the filter (5) can be controlled;
the control unit, for the purpose of filling and venting the patient module, is designed to close the first controllable valve (21), which is open after the start of the filling process for the venting line of the filter (5), after the upper filling level sensor (10) in the reservoir (2) responds in order to control active filling of the filter (5); and
an air cushion is created in the filter by means of the closed first controllable valve (21) and the thus closed venting line, which air cushion dampens inflowing filling liquid in its flow behaviour such that the filter is only partially filled and an upper region of the filter element remains unwetted by the filling liquid.

2. System according to claim 1, wherein the system is adapted in order to record the process of filling and venting and therefore log said process in such a way that it can be read out.

3. System according to claim 2, wherein the system has a memory unit in which data such as the switch positions of the controllable valves, filling levels of the reservoir, on and off times, the response of the air bubble sensor and other parameters such as pressure and flow can be stored.

4. System according to either claim 2 or claim 3, wherein the system further comprises pressure and flow sensors which are integrated into the blood circulation system.

5. System according to claim 1, wherein the blood pump is arranged below the maximum filling level of the reservoir 2 such that the blood pump 6 can be filled by means of gravity and there is therefore always liquid in the blood pump 6 during the venting process.

6. System according to claim 1, wherein the blood pump (6) is a centrifugal pump.

7. System according to claim 1, wherein:
the air bubble sensor (30) is arranged downstream of the filter (5) and activates a subsequent fast-closing clip (20) when a bubble is detected, wherein the fast-closing clip (20) closes an arterial line (42) and opens a hose clip (24) for a bypass (40) when an air bubble is detected by the bubble sensor, such that the liquid is pumped around in the circuit until the detected bubble is no longer in the blood circulation system of the patient module (1).

## Revendications

1. Système pour le traitement extracorporel du sang, lequel système est réalisé en deux parties et se compose d'un module de commande doté d'un module pour patient, dans lequel :
le module de commande comporte des colliers de serrage automatiques ou des soupapes commandables destinées aux conduites de fluide, un entraînement de pompe à sang et un système électronique d'évaluation et de commande (302) ;
le module pour patient (1, 1a), lequel module pour patient est un dispositif de traitement extracorporel du sang, comporte une circulation sanguine comprenant une ligne veineuse (15), un réservoir (2), un oxygénateur (3), un détecteur de bulles d'air (30), un filtre (5), une ligne artérielle (42) et une pompe à sang (6) ;
le réservoir (2) est équipé d'un capteur de niveau de remplissage supérieur (10) ;
le filtre (5) comprend un élément filtrant et une ligne de ventilation fixée à une partie supérieure du filtre, la ligne de ventilation pouvant être fermée par une première soupape commandable (21) disposée dans la ligne de ventilation ;
le système comprend une unité de commande (302) destinée au remplissage et à la ventilation entièrement automatisée d'un module pour patient selon l'invention ;
un côté supérieur du filtre (5) est plus élevé que le capteur de niveau de remplissage supérieur (10) du réservoir, permettant ainsi de commander un remplissage partiel du filtre (5) ;
l'unité de commande, destinée au remplissage et à la ventilation du module pour patient, est configurée pour fermer la première soupape commandable (21), laquelle soupape commandable, après le démarrage du processus de remplissage, est ouverte pour la ligne de ventilation du filtre (5), après la réponse du capteur de niveau supérieur (10) dans le réservoir (2), afin de commander le remplissage actif du filtre (5) ; et
un coussin d'air est créé dans le filtre par la première soupape commandable (21) fermée et la ligne de ventilation ainsi fermée, lequel coussin d'air amortit le comportement d'écoulement du liquide de remplissage entrant, de sorte que le filtre ne soit rempli que partiellement et qu'une zone supérieure de l'élément filtrant reste non mouillée par le liquide de remplissage.

2. Système selon la revendication 1, dans lequel le système est adapté pour enregistrer le processus de remplissage et de ventilation et le consigner ainsi sous une forme lisible.

3. Système selon la revendication 2, dans lequel le système comprend une unité de stockage dans laquelle des données telles que les positions de commutation des soupapes commandables, les niveaux de remplissage du réservoir, les temps d'arrêt et de mise en marche, la réponse du capteur de bulles d'air et d'autres paramètres tels que la pression et l'écoulement peuvent être stockés.

4. Système selon la revendication 2 ou 3, le système comprenant en outre des capteurs de pression et d'écoulement intégrés dans la circulation sanguine.

5. Système selon la revendication 1, dans lequel la pompe à sang est disposée en dessous du niveau maximal du réservoir 2, de sorte que la pompe à sang 6 puisse être remplie par gravité et qu'il y ait toujours du liquide dans la pompe à sang 6 pendant le processus de ventilation.

6. Système selon la revendication 1, dans lequel la pompe à sang (6) est une pompe centrifuge.

7. Système selon la revendication 1, dans lequel :
le capteur de bulles d'air (30) est disposé en aval du filtre (5) et active une pince à action rapide (20) suivante lorsque des bulles d'air sont détectées, la pince à action rapide (20) fermant une ligne artérielle (42) lorsqu'une bulle d'air est détectée par le capteur de bulles d'air et ouvrant un collier de serrage (24) pour une dérivation (40), de sorte que le liquide soit pompé en rond dans un circuit jusqu'à ce que la bulle d'air détectée ne soit plus dans la circulation sanguine du module pour patient (1).
